**Europäisches Patentamt**

**European Patent Office**

**Office européen des brevets**

(19)

(11) Publication number: **0 089 709**

**A1**

(12) **EUROPEAN PATENT APPLICATION**

(21) Application number: **83200320.6**

(22) Date of filing: **07.03.83**

(51) Int. Cl.³: **C 10 M 3/20**
**C 10 M 1/26**

(30) Priority: **19.03.82 IT 2026482**

(43) Date of publication of application:
**28.09.83 Bulletin 83/39**

(84) Designated Contracting States:
**BE CH DE FR GB LI NL SE**

(71) Applicant: **AGIP PETROLI S.p.A.**
**Via Laurentina 449**
**I-00142 Roma(IT)**

(71) Applicant: **ANIC S.p.A.**
**Via Ruggero Settimo, 55**
**I-90139 Palermo(IT)**

(72) Inventor: **Koch, Paolo**
**Via Palmiro Togliatti, 18**
**I-20077 Melegnano (Milan)(IT)**

(72) Inventor: **Romano, Ugo**
**Via XXV Aprile 10**
**I-20059 Vimercate Milan(IT)**

(74) Representative: **Roggero, Sergio et al,**
**Ing. Barzanò & Zanardo Milano S.p.A. Via Borgonuovo**
**10**
**I-20121 Milano(IT)**

(54) **Synthesis of higher alcohol carbonates and their use as synthetic lubricants.**

(57) Organic carbonic acid esters of higher alcohols which can be used in formulating lubricants for internal combustion engines and/or industrial machines, and the process for their preparation.

EP 0 089 709 A1

CASE 0089709

This invention relates to synthetic products, and in particular to organic carbonic esters of higher alcohols which can be used in formulating lubricants for internal combustion engines and/or industrial machines.

The use of synthetic components is known to enable multigrade lubricants to be prepared more easily, by overcoming the drawbacks which are often encountered when using only natural bases (the presence of extremely fluid mineral fractions introduced in order to attain the required viscosities at low temperature, the need for high percentages of viscosity index improvement additives etc.). In order to be used advantageously for this purpose, the synthetic base must possess suitable characteristics. For use in automobile engines, the product must have low volatility in relation to its viscosity. The viscosity-temperature characteristics must also be such as to allow easy cold starting, while at the same time ensuring good lubrication at maximum operating temperature. Finally, the synthetic base must have high thermal stability and resistance to oxidation, and good lubricating power.

In the case of oils for lubricating industrial machines (refrigerant oils, hydraulic oils, compressor oils etc.), the synthetic base must mainly have a low freezing point and good film adhesion to the mechanical parts to be lubricated. In addition, any products deriving from its thermal, hydrolytic or oxidation decomposition must not have such characteristics as to be aggressive or corrosive to metals.

A lubricant formulation has therefore been sought which would obviate the aforesaid drawbacks, while at the same time possessing all the characteristics necessary for its application both in internal combustion engines and in industrial machines.

It has been found that carbonic acid esters can satisfy the aforesaid requirements.

In this respect, the choice of the alcohol enables carbonic esters to be obtained with widely different rheological characteristics, and thus able to cover numerous fields of application.

The carbonic acid esters of higher alcohols have been synthesised by transesterification with higher alcohols starting from dimethylcarbonate (DMC) and/or analogous esters of low boiling alcohols prepared by the ANIC process from alcohols and carbon monoxide. (Italian patent No. 898077 and the U.K. Patent N°1.574.188 granted on November 19, 1980).

The higher alcohols used are mainly alcohols having an average molecular weight of between 100 (cyclohexanol) and 270 (octadecyl alcohol).

The transesterification reaction can be carried out either in the homogeneous or heterogeneous phase, the catalysts used being bases such as alkaline alcoholates, alkaline hydrates, aliphatic amines (for example trialkylamine) or heterocyclic amines (for example pyridine or N-methylimidazole).

The reaction is carried out at a temperature of between 20 and 300°C, and preferably between 100 and 200°C.

The reaction can be carried out at a pressure either greater or less than atmospheric, according to the volatility of the reactants and the products of reaction, and the pressure can in fact vary from 10 atm. absolute to 0.1 mmHg absolute.

The reaction system is equally suitable for use in a discontinuous process (in a batch reactor), a semi-continuous process (with distillation of the low-boiling products such as methanol and ethanol), or in a totally continuous process in a distillation column.

If operating in the homogeneous phase, hydrocarbon solvents (hexane, heptane, benzene, toluene) or ethers (dioxane, tetrahydrofuran) can be used, possibly chosen so as to form azeotropes with the low-boiling alcohols produced during the reaction.

If operating in the heterogeneous phase, the catalyst can consist of a basic ion exchange resin, or an alcoholate impregnated on an inert support.

From the aforegoing, it is apparent that as the reaction is reversible, it proceeds in the required direction by removing the low-boiling alcohol by fractionation.

If operating as a batch process, on termination of the reaction the unreacted alcohol is removed, whereas the catalyst can either be removed by filtration, washing or rectification (amine) or can be left in the product, especially if using high-boiling amines.

The fact that the system is very suitable for continuous synthesis in a column can be a considerable technical advantage, because it is known that such methods lead to considerable reductions in reaction time, investment cost and operating costs.

The alcohols used are generally primary and mainly linear, but these have also been used in mixture with branched alcohols which have the advantage of lowering the product freezing point.

The product for use in the lubricant field can be utilised either alone or in mixture, and is characterised chemically on the basis of the alkyl chains present, which depend on the type of alcohol mixtures used, and on certain physical properties.

The products are however completely monomeric.

As stated, by suitably choosing the starting alcohol it is possible to obtain a series of products with the most suitable characteristics for use in the various fields of lubrication, and to satisfy the most varied requirements.

The rheological characteristics of these products vary with the alcohol structure. Linearity leads to products with a high viscosity index and freezing point, and branching leads to products with a low viscosity index and freezing point.

Esters of branched alcohols (iso $C_{10}$, iso $C_{13}$) have a low freezing point and low viscosity index, while esters of linear alcohols (n-dodecyl) or partially linear alcohols have high freezing points and good viscosity indices.

An ester obtained from a mixture of linear and branched alcohols has intermediate characteristics.

The carbonic esters of the present invention can be used as lubricants for internal combustion engines either as such, or by formulating them in various proportions (1-50% by weight) with mineral oils. These bases, whether completely or partially synthetic, can be dosed with the known commercial components for satisfying the necessary engine application requirements.

The excellent lubricating properties of these carbonic esters allow them to be also widely used in the industrial lubrication sector, such as in hydraulic transmission oils, compressor oils etc., in which exceptional adherence of the lubricating film to the metal is required under extremely severe conditions.    These characteristics are demonstrated by the better tribological behaviour of carbonic esters compared with that of the other synthetic or mineral bases mentioned hereinafter.

The physical characteristics and chemical behaviour of the carbonic esters also indicate a performance which is distinctly better than that of carboxylic esters with equal rheological properties.    In this respect, the results of thermal, hydrolytic and thermo-oxidation stability tests on carbonic esters and on a carboxylic comparison ester of equal viscosity are given hereinafter.

The carbonic esters are found to be hydrolytically, oxidationally and thermally more stable than the carboxylic ester, and whereas the increase in acidity of the carbonic ester is due to the weak inorganic acid $H_2CO_3$ formed by decomposition, the increase in acidity due to the carboxylic ester is due to the presence of strong highly corrosive organic acids.

EXAMPLES

The examples given hereinafter illustrate the present invention, and the characteristics of the products described in the examples when compared with the characteristics of conventional products (such as organic acid esters) demonstrate the advantages obtained.

Synthesis of carbonic acid esters

The synthesis apparatus consisted of a three-neck flask having a volume of 1000-5000 cc, which was jacketed and temperature-controlled by means of an externally circulating fluid, and was surmounted by a perforated plate distillation column (⌀ 30 mm) with a liquid dividing head, and was fitted with a magnetic agitator and thermometer.

The carbonate of the low-boiling alcohol, the higher alcohol and the catalyst consisting of an organic or inorganic strongly basic compound, were fed into the flask.

The reaction was carried out in an inert atmosphere by heating the mixture to boiling point and removing as overheads the low-boiling alcohol which formed.  If considered useful, the reaction can also be carried out in the presence of an inert solvent which can form a minimum azeotrope with the low-boiling alcohol so as to facilitate its removal.  At the end of the reaction, the catalyst can be separated from the reaction mixture by filtration, by washing with water, or by neutralisation.

The reaction product can be the mixed ester containing both the alcohols, namely the high and low-boiling alcohols, or the diester of the high-boiling alcohol, according to the molar   ratio of the carbonate to the reactive alcohol in the feed.

If a mixture of alcohols is fed to the reaction, these react to give rise to a mixture of carbonic esters.

EXAMPLE 1

118 g (1 mole) of diethylcarbonate, 391.2 g (2.1 moles) of n-dodecyl alcohol and 1 g of a solution of 10% sodium ethylate in ethanol are reacted together.

The reaction is initially carried out at atmospheric pressure, but vacuum is applied as the reaction proceeds, so as to maintain a bottom temperature of not greater than $160^{\circ}C$.

At the end of the reaction, 86 g of an overhead fraction had been obtained consisting of ethanol (90.2%) and diethyl carbonate (9.8%).

The product (424 g) consisted of di-n-dodecyl carbonate containing small quantities of ethyl-n-dodecyl carbonate and n-dodecanol. At this point, after filtering off the catalyst, the vacuum was increased to 0.3 mmHg, and the n-dodecanol and mixed carbonate were distilled off.

The residue (344 g) consisted of di-n-dodecyl carbonate containing 1.5% of ethyl-n-dodecyl carbonate, the distillate (78 g) consisting essentially of n-dodecanol and mixed carbonate with small quantities of ethanol and di-n-dodecyl carbonate (Table 1).

EXAMPLES 2-9

The syntheses of a series of products obtained by an analogous procedure to that of example 1 are reported in Table 1.

EXAMPLE 10

1704 g (12 moles) of diallyl carbonate, 416 g (2 moles) of a mixture of $C_{12}$-$C_{15}$ oxo-alcohols, and 8 g of a solution of 30% sodium methylate in methanol were reacted together.

The reaction was carried out at 150 mmHg for a time of one hour. 128 g of distillate were obtained containing 118 g of diallyl alcohol and 10 g of diallyl carbonate.

The bottom product, containing about 0.4% of alcohols, was washed with water in order to eliminate the catalyst.

The excess diallyl carbonate was then distilled off under reduced pressure, to obtain an overhead product of 1500 g of diallyl carbonate, and a residue of 560 g consisting of 0.6% diallyl

carbonate, 1.0% alcohols, 91.6% mixed carbonates and 6.8% oxo-alcohol carbonates.

EXAMPLES 11-12

Examples of mixed carbonates obtained by a procedure analogous to Example 10 are reported in Table 2.

The characteristics of some of the obtained products are reported in Table 3.

LABORATORY EVALUATIONS

Table 4 shows the laboratory evaluations carried out on the $C_{12}-C_{15}$ oxo-alcohol ester of Example 5 (lubricant A), and also, for comparison purposes, the results of analogous tests carried out on a carboxylic comparison ester prepared by esterification of trimethylolpropane with n-heptanoic acid (lubricant B) and having the following rheological characteristics: $V_{40^{\circ}C}$ 16.53 cSt, $V_{100^{\circ}C}$ 3.95 cSt, V.I. 139.

TRIBOLOGICAL TESTS

The tribological characteristics of the fluids considered herein were determined by tests carried out with the LFW-1 and SRV tribometers.

The operating conditions used for tests with the LFW-1 machine were those pertaining to the ASTM 2714-14 method, namely: ring speed 72 rpm, load 68 kg, duration 5000 cycles.

As required by this method, determinations were made of the friction force after 4500 cycles, and the width of the wear impression in the block and the loss of weight of the block at the end of the test.

In the SRV (Schwingungs-Reib-und-Verschleisstester) machine the test piece consists of two shoes, a lower and an upper, between which the lubricant under test is placed.    These are loaded one against the other with a variable force, and the upper body is moved tangentially to the lower shoe with a horizontal reciprocating movement which is preferably variable and mostly sliding.

The load, which can vary from 0 to 120 kg, is applied by means of an electromagnet operating on alternating current which can have an amplitude of 50 to 1200 $\mu$ and a frequency of 15 to 400 Hz, and produced by a moving coil device.

The machine enables a determination to be made of the friction force created between the two shoes as a consequence of the imposed movement, and allows continuous recording of the friction coefficient by using a piezoelectric device.

The geometry of the contact zone varies according to the shape of the two shoes which make up the test piece, and can be represented by a point, by a line or by a surface.

In the case of the measurements reported herein, test pieces were used consisting of a steel ball (mobile part) which was loaded against a flat surface constituting the base of a steel cylinder (fixed part).

The test conditions used were the following:  load 150 N, amplitude 1000 $\mu$, frequency 50 Hz, temperature $100^{\circ}$C, duration 1 hour.

In addition to the friction coefficient, at the end of the test an intensity measurement was taken expressed as the width of the wear indentation on the ball.

The carbonic ester of Example 5 (lubricant A), the carboxylic ester formed from trimethylolpropane and n-heptanoic acid (lubricant B) and a paraffin mineral oil type SN 150 (lubricant C) were all subjected to these two tribological evaluations.

The results are given in Tables 5 and 6.

ENGINE TESTS

Comparison tests were made on a lubricant composition containing 15% of higher alcohol carbonates (lubricant A) according to the present patent application, and a lubricant composition containing 15% of a carboxylic acid ester prepared by esterification of trimethylolpropane with n-heptanoic acid and having the following rheological characteristics: $V_{40°C}$ 16.53 cSt, $V_{100°C}$ 3.95 cSt, V.I. 13.9 (lubricant B).

A commercial pack of engine additives was added to the lubricant compositions to a concentration of 8%.

The following engines were used for this purpose:

- Petter W1

- Mercedes 240 D

- Alfa Romeo "Alfetta".

- The method using the Petter W1 petrol engine knowingly demonstrates the anticorrosive properties of the oil (evaluation of the loss of weight of the connecting rod copper bearing) because of the high temperatures existing in the sump and at other "hot" points of the engine.

The procedure is described in pamphlet CEC L-02-A-78, and forms part of the European Sequence for oil classification at the CCMC level.

- The method using the Mercedes 240 diesel engine emphasises the wear conditions at the cam-rocker arm coupling. The wear which arises is mainly of the corrosive type (SAE paper 811226), and previous experiments have shown that this phenomenon can occur in the presence of oil containing ester bases. The procedure is described in pamphlet CEC L-17-A-78, and is also inserted in the European Sequence required by manufacturers.

- The method using the Alfa Romeo "Alfetta" engine mainly evaluates the "lead paste" deposits which form on the piston, and in addition because of the high power and fuel consumption imposed on the engine during its operation on the test bed, useful information is gained regarding the lacquer deposits on the critical piston zones and the carbon deposit in the first groove. These data represent a judgement on the thermal stability of the oil when used in an engine which is representative of those in circulation at the present time.

Table 7 gives the values for the two oils in a 36 hour test carried out on the Petter W1 engine.

Table 8 gives values for the two oils in a 50 hour test carried out on the Mercedes 240 D engine.

Table 9 gives the values for the two oils in a 30 hour test carried out on the Alfetta engine.

TABLE 1 - Tests involving the preparation of higher alcohol carbonates starting from DMC or DEC and higher alcohols

| Example No. | Higher alcohol | Carbonylating agent | Molar ratio DMC or DEC: higher alc. | Catalyst (1), % total feed by weight | Reaction temperature ($^oC$) | Reaction time (hours) | % conversion carbonylating agent | % yield dialkylcarbonate on carbonylating agent |
|---|---|---|---|---|---|---|---|---|
| 1 | n-dodecyl | DEC | 0.48 | 0.20 | 146-160 | 2.5 | 96 | 82 |
| 2 | cyclohexanol | DEC | 0.48 | 0.53 | 120-157 | 2.0 | 95 | 84 |
| 3 | $C_{16}$-$C_{19}$ oxo-alcohols | DEC | 0.48 | 0.20 | 155-183 | 3 | 95 | 89 |
| 4 | isodecyl | DEC | 0.48 | 0.30 | 110-150 | 2.7 | 99 | 96.1 |
| 5 | 25 mol% isodecyl + 75 mol% $C_{12}$-$C_{15}$ oxo-alcohols | DEC | 0.48 | 0.30 | 115-150 | 2.7 | 98.8 | 91.5 |
| 6 | isotridecyl | DEC | 0.48 | 0.30 | 117-153 | 3.3 | 97.8 | 87.2 |
| 7 | 50 mol% isotridecyl + 50 mol% $C_{12}$-$C_{15}$ oxo-alcohols | DEC | 0.48 | 0.30 | 119-153 | 4.2 | 97.2 | 90.3 |
| 8 | $C_{12}$-$C_{15}$ oxo-alcohols | DMC | 0.56 | 0.30 | 96-190 | 5.5 | 76.5 | 70.5 |
| 9 | n-octyl | DMC | 0.56 | 0.40 | 84-166 | 6 | 80.0 | 75.6 |

(1) 30 weight% solution of $CH_3ONa$

TABLE 2 - Tests involving the preparation of mixed ethyl (or cyclohexyl or allyl) and higher alcohol carbonates

| Example No. | Higher alcohol | Carbonylating agent | Molar ratio carbonyl. agent: higher alcohol | Catalyst (1) % total feed by weight | Reaction temp. ($^{o}$C) | Pressure (mmHg) | Reaction time (hours) | % yield mixed carbon- ates | % yield higher alcohol carbon- ates |
|---|---|---|---|---|---|---|---|---|---|
| 10 | $C_{12}-C_{15}$ oxo-alcohols | diallyl- carbonate | 12.0/2.0 | 0.4 | 99-121 | 150 | 0.66 | 86.9 | 13.1 |
| 11 | $C_{16}-C_{19}$ oxo-alcohols | dicyclohexyl- carbonate | 7.5/1 | 0.6 | 168-176 | 100-10 | 1.0 | 80.0 | 20 |
| 12 | oleyl | diethyl- carbonate | 5.4/1 | 0.5 | 109-133 | 760 | 1.5 | 91.8 | 8.2 |

(1)   30 weight% solution of $CH_3ONa$

TABLE 3 - Characteristics of higher alcohol carbonates

| Example No. | Carbonates | sp.gr.a. at $20^{\circ}C$ | cSt at $40^{\circ}C$ | cSt at $100^{\circ}C$ | V.I. | Pour point ($^{\circ}C$) | $\%H_2O$ | M.W. |
|---|---|---|---|---|---|---|---|---|
| 1 | n-dodecyl | 0.879 | 12.2 | 3.4 | 169 | +5 | - | 398.7 |
| 3 | $C_{16}$-$C_{19}$ alcohols | 0.866 ($30^{\circ}C$) | - | 5.7 | -- | +24 | 0.005 | 542.0 average |
| 4 | isodecyl | 0.888 ($25^{\circ}C$) | 9.4 | 2.5 | 86 | -57 | 0.005 | 342.6 |
| 5 | mixed, 25 mol% isododecyl, 75 mol% $C_{12}$-$C_{15}$ oxo-alcohols | 0.876 ($25^{\circ}C$) | - | - | - | +7 | 0.012 | 417.0 average |
| 6 | isotridecyl | 0.889 | 19.8 | 3.9 | 81 | -42 | 0.016 | 426.6 |
| 7 | mixed, 50 mol% isotridecyl, 50 mol% $C_{12}$-$C_{15}$ oxo-alcohols | 0.878 ($24^{\circ}C$) | 16.9 | 3.7 | 113 | -15 | 0.021 | 434.3 average |
| 8 | $C_{12}$-$C_{15}$ alcohols | 0.876 | 15.7 | 3.8 | 144 | +9 | 0.005 | 442.0 |

## TABLE 4 - LABORATORY EVALUATIONS

| | Synthetic lubricant | Δviscosity % cSt | NN oil mg KOH/g before | NN oil mg KOH/g after | Acidity tot. water mg KOH | Cu corrosion Δweight mg/g | Cu corrosion colour | Lubricant weight loss % |
|---|---|---|---|---|---|---|---|---|
| HYDROLYTIC STABILITY | A | -0.05 | 0 | 0.63 | 2.80 | - | 3A | - |
| 93°C x 48 hours | B | -0.85 | 0.11 | 0.17 | 5.61 | - | 2C | - |
| OXIDATION STABILITY | A | +18.1 | 0 | 3.61 | - | 0.91 | 1A | 1.8 |
| 175°C x 24 hours | B | +35 | 0.11 | 4.43 | - | 1.89 | 1A | 1.2 |
| THERMAL STABILITY | A | -0.5 | 0 | 0.95 | | | | |
| 270°C x 24 hours | B | +0.49 | 0.11 | 1.47 | | | | |

TABLE 5α LFWI-1 Test

|  | Wear width (mm) |
| --- | --- |
| Lubricant A | 1.27 |
| Lubricant B | 4.28 |
| Lubricant C | 2.83 |

TABLE 6  SRV Test

|  | Wear diameter (mm) | Coeff. of friction MIN. | MAX. |
| --- | --- | --- | --- |
| Lubricant A | 0.34 | 0.040 | 0.044 |
| Lubricant B | 0.68 | 0.051 | 0.071 |
| Lubricant C | 0.46 | 0.073 | 0.095 |

TABLE 7  Engine Test Petter W1 engine

| OIL | BEARING WEIGHT LOSS (mg) | VISCOSITY INCREASE at 40°C (%) | CCMC LIMITS |
| --- | --- | --- | --- |
| A | 14.7 | 37.6 | 25 mg max |
| B | 17.3 | 30.3 | 100% max |
|  | 11.9 | 40.6 |  |

TABLE 8   Engine Test Mercedes 240D engine

| OIL | AVERAGE WEAR 8 CAMS (μm) | MAX. WEAR OF 1 CAM (μm) | ACCEPTABILITY LIMIT |
|-----|--------------------------|-------------------------|---------------------|
| A   | 0.31                     | 1                       |                     |
|     | 4.12                     | 7                       | Average = 15 μm     |
| B   | 5.87                     | 13                      | Max = 30 μm         |

TABLE 9   Engine Test Alfetta engine

| OIL | TOTAL Pb PASTE COUNT (MAX 100) | TOTAL LACQUER COUNT (MAX 100) | CARBON 1st GROOVE (%) |
|-----|--------------------------------|-------------------------------|-----------------------|
| A   | 98.9                           | 79.4                          | 11.1                  |
| B   | 100                            | 71.9                          | 17.7                  |
|     | 99.7                           | 81.5                          | 19.9                  |

CLAIMS:

1. A lubricant composition containing one or more higher alcohol carbonates.

2. A lubricant composition containing mineral oil, one or more additives, and one or more higher alcohol carbonates.

3. A composition as claimed in the preceding claim, wherein the higher alcohol carbonate is present in a quantity of between 1% and 50% by weight.

4. A process for preparing higher alcohol carbonates, by the transesterification of carbonates of low boiling alcohols with higher alcohols.

5. A process as claimed in the preceding claim, wherein the higher alcohol has a molecular weight of between 100 and 270.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X,Y | US-A-3 642 858 (L.K. FREVEL)<br>* Claim 1; column 3, lines 61-62; column 2, lines 52-63 *<br>--- | 1,5 | C 10 M 3/20<br>C 10 M 1/26 |
| X | US-A-2 758 975 (D.L. COTTLE)<br>* Claims 1-4 *<br>--- | 1,5 | |
| X,Y | US-A-2 387 999 (A.T. KNUTSON)<br>* Claims 1,8,11 *<br>--- | 1-3,5 | |
| Y | US-A-2 915 529 (J.A. BELL)<br>* Claim 1 *<br>----- | 4 | |

TECHNICAL FIELDS SEARCHED (Int. Cl. ³)

C 10 M
C 07 C

The present search report has been drawn up for all claims

| Place of search<br>THE HAGUE | Date of completion of the search<br>16-06-1983 | Examiner<br>ROTSAERT L.D.C. |
|---|---|---|